# EUROPEAN PATENT APPLICATION

(11) **EP 3 974 016 A1**
(43) Date of publication of application: **30.03.2022**
(21) Application number: 20854221.7
(22) Date of filing: 18.08.2020
(51) Int. Cl.: A61M 25/01, A61M 25/14

(54) **CATHETER**

(30) Priority: 20.08.2019 JP 2019150512
(71) Applicant: Goodman Co., Ltd., Aichi 460-0008 (JP)
(72) Inventor: HIROTA, Toru, Seto-shi, Aichi 489-0976 (JP); MIYAKE, Takamasa, Seto-shi, Aichi 489-0976 (JP); OGAWA, Keisuke, Seto-shi, Aichi 489-0976 (JP); OTA, Mitsuhiro, Seto-shi, Aichi 489-0976 (JP); SATO, Mitsuhiko, Seto-shi, Aichi 489-0976 (JP)
(74) Representative: Prüfer & Partner mbB Patentanwälte · Rechtsanwälte
(86) International application number: PCT/JP2020/031052
(87) International publication number: WO 2021/033673

(57) **Abstract**

Provided is a balloon catheter comprising: an outer tube 11; an inner tube 12 which is inserted into a lumen 11a of the outer tube 11 and the base-end portion of which is bonded to the outer tube 11; and a core wire 30 which is inserted into the lumen 11a of the outer tube 11 and is extended further toward the leading-end side than the base-end portion of the inner tube 12. In an overlapping range 35 in which the inner tube 12 and the core wire 30 overlap in the axial direction, a wall portion 37 is disposed at an intermediate position in said axial direction. A hole part 41 through which the inner tube 12 is inserted and a hole part 42 through which the core wire 30 is inserted are formed in the wall portion 37. The inner tube 12 is secured to the outer tube 11 via the wall portion 37, and the core wire 30 is not secured to the wall portion 37.

## Description

### Cross-Reference to Related Application

The present application is based on Japanese Patent Application No. 2019-150512 filed on August 20, 2019, the contents of which are incorporated herein by reference.

### Technical Field

The present disclosure relates to a catheter.

### Background Art

In the related art, balloon catheters have been used for remedies such as PTA (percutaneous transluminal angioplasty) and PTCA (percutaneous transluminal coronary angioplasty). Such a balloon catheter is configured to include an outer tube, an inner tube inserted into the outer tube, and a balloon provided on a tip-end side of each tube.

The balloon is joined to a tip-end portion of the outer tube. A lumen of the outer tube is a fluid lumen that allows a fluid to be distributed therethrough, and the balloon is expanded or contracted by causing a compressed fluid to be distributed through the lumen of the outer tube.

The inner tube is provided in a state in which the inner tube further extends on the tip-end side beyond the outer tube, and the extending part extends on the tip-end side beyond the balloon through the inside of the balloon. The inner tube has a tip-end portion joined to a tip-end portion of the balloon. A lumen of the inner tube is a guide wire lumen into which a guide wire is inserted, and when a balloon catheter is inserted into a body, a guide wire introduced into the body in advance is inserted into the lumen of the inner tube, and the balloon catheter is then introduced into the body in the inserted state.

As a method of inserting a guide wire, a so-called RX type in which the guide wire is guided to the outside at a midpoint position of the outer tube in an axial line direction is known. In an RX-type balloon catheter, a guide wire port is formed to penetrate through a circumferential wall portion of the outer tube at a midpoint of the tube in the axial line direction, and the lumen of the inner tube is opened to the outside of the catheter through the guide wire port. In this case, a base-end portion of the inner tube is joined to a midpoint position of the outer tube in the axial line direction, specifically, a peripheral edge portion of the guide wire port. Such an RX-type catheter is adapted such that the guide wire introduced into the inner tube from the tip-end portion of the tube is guided to the outside of the catheter through the guide wire port.

Incidentally, such an RX-type balloon catheter may be provided with a core wire for the purpose of enhancing rigidity or the like (see Patent Literature 1, for example). The core wire is provided to be inserted into the lumen of the outer tube and is secured, at its base-end portion, to a hub. Also, the core wire is provided in a state in which the core wire extends on the tip-end side beyond the guide wire port, for example. In this case, it is possible to apply rigidity to the balloon catheter up to the tip-end side and thereby to improve transmissibility of a force when the balloon catheter is introduced into a body.

### Citation List

### Patent Literature

Patent Literature 1: Japanese Patent Laid-Open No. 2008-125897

### Summary of Invention

### Technical Problem

Here, according to the aforementioned configuration in which the core wire is provided to extend on the tip-end side beyond the guide wire port, the core wire and the inner tube are disposed to be aligned inside (lumen) the outer tube on the tip-end side beyond the guide wire port. Therefore, a case in which the inner tube and the core wire are entangled with each other, for example, the inner tube twists around the core wire, when the balloon catheter is introduced into a body is assumed. In such a case, there is a concern that a problem such as degradation of insertability of the guide wire in the inner tube may occur.

Note that such a problem is a problem that is not limited to the RX-type balloon catheter and may occur similarly in other RX-type catheters.

The present disclosure was made in view of the aforementioned circumstances, and a main object thereof is to provide a catheter capable of restraining an inner tube and a core wire from being entangled inside an outer tube.

### Solution to Problem

In order to solve the aforementioned problem, a catheter according to a first disclosure includes: an outer tube that includes a lumen therein; an inner tube that is inserted into the lumen of the outer tube and has a base-end portion joined to an intermediate position of the outer tube in an axial line direction; and a core wire that is inserted into the lumen of the outer tube and is provided to extend on a tip-end side beyond the base-end portion of the inner tube, and a securing portion that secures the inner tube or the core wire to the outer tube is provided at an intermediate position of an overlapping range, in which the inner tube and the core wire overlap each other in the axial line direction, in the axial line direction.

According to the present disclosure, the inner tube and the core wire are inserted into the lumen of the outer tube. The inner tube has the base-end portion joined to the intermediate position of the outer tube in the axial line direction, and the core wire is provided in a state in which the core wire is caused to extend on the tip-end side beyond the base-end portion of the inner tube. In this case, the overlapping range in which the inner tube and the core wire overlap each other in the axial line direction is present inside (lumen) of the outer tube.

In the present disclosure, the securing portion that secures the inner tube or the core wire to the outer tube is provided at the intermediate position of the overlapping range in the axial line direction in such a configuration. In this case, it is possible to restrain displacement of the inner tube or the core wire inside the outer tube. It is thus possible to restrain the inner tube and the core wire from being entangled inside the outer tube.

In the catheter according to a second disclosure, the securing portion is adapted to secure the inner tube to the outer tube, in the first disclosure.

Generally, the core wire has higher rigidity than the inner tube. Therefore, there is a concern that followability when the catheter is inserted into a curved blood vessel may be degraded in a case in which the core wire is secured to the outer tube. In this regard, according to the present disclosure, the inner tube out of the inner tube and the core wire is secured to the outer tube, and it is thus possible to obtain the advantage of the first disclosure while avoiding such a concern.

The catheter according to a third disclosure further includes: a separating portion that is interposed between the inner tube and the core wire to separate the inner tube and the core wire, in the first or second disclosure.

According to the present disclosure, the inner tube and the core wire are separated by the separating portion interposed therebetween, and it is thus possible to further restrain the inner tube and the core wire from being entangled.

The catheter according to a fourth disclosure further includes: a wall portion that is provided to section the lumen in the axial line direction, a hole portion into which the inner tube is inserted and a hole portion into which the core wire is inserted are formed in the wall portion, any one of the inner tube and the core wire is secured to the outer tube via the wall portion serving as the securing portion while the other one of the inner tube and the core wire is not secured to the wall portion, and the separating portion is formed by a part between the hole portions in the wall portion, in the third disclosure.

According to the present disclosure, the wall portion is provided to section the lumen of the outer tube in the axial line direction, and each of the inner tube and the core wire is inserted into each hole portion formed in the wall portion. In such an inserted state, any one of the inner tube and the core wire is secured to the outer tube via the wall portion while the other one of the inner tube and the core wire is not secured to the wall portion. Also, the part between the hole portions in the wall portion is interposed between the inner tube and the core wire and serves as the separating portion. In this case, it is possible to use the wall portion as the separating portion in addition to using the wall portion as the securing portion securing the one of the inner tube and the core wire. It is thus possible to suitably restrain tangling of the inner tube and the core wire with a relatively simple configuration.

In the catheter according to a fifth disclosure, the inner tube as the one of the inner tube and the core wire is secured to the outer tube via the wall portion while the core wire as the other one of the inner tube and the core wire is not secured to the wall portion, in the fourth disclosure.

According to the present disclosure, it is possible to obtain both the advantage of the second disclosure and the advantage of the fourth disclosure at once.

In the catheter according to a sixth disclosure, the lumen is a fluid lumen through which a fluid flows, and the hole portions have long hole shapes, in the fourth or fifth disclosure.

According to the present disclosure, the hole portions provided in the wall portion have the long hole shapes, and it is thus possible to secure wide non-insertion regions that are not used for insertion in the hole portions in a state in which the core wire and the inner tube are inserted into the hole portions. In this case, the non-insertion regions in the hole portions can be used to allow the fluid to pass therethrough when the lumen of the outer tube is used as the fluid lumen through which the fluid flows. Therefore, there is no need to separately provide a hole portion for the fluid in the wall portion to allow the fluid to pass therethrough, and it is possible to simplify the configuration of the wall portion.

In the catheter according to the seventh disclosure, the hole portion into which the other one of the inner tube and the core wire that is not secured to the wall portion is inserted out of the hole portions has a long hole shape, in the sixth disclosure.

In the aforementioned fourth disclosure, any one of the inner tube and the core wire is secured to the wall portion while the other one of the inner tube and the core wire is not secured to the wall portion. With such a configuration, it is desirable for the shape of the hole portion for the one of the inner tube and the core wire to have a shape (a circular shape, for example) in accordance with a sectional shape of the one of the inner tube and the core wire since it is necessary to firmly secure the one of the inner tube and the core wire to the wall portion in a state in which the one of the inner tube and the core wire is inserted into the hole, while there is no need to form the hole portion to have such a shape for the other one of the inner tube and the core wire since the other one of the inner tube and the core wire is not secured. Thus, in view of such a point, (only) the hole portion into which the other one of the inner tube and the core wire is inserted out of the hole portions is formed into the long hole shape in the present disclosure. In this case, a configuration that is preferable in practice can be achieved to realize the aforementioned sixth disclosure.

In the catheter according to an eighth disclosure, the core wire has, on a tip-end side, a tapered region that is formed into a tapered shape to be narrowed toward the tip end, and the tapered region is disposed over the entire overlapping range, in any one of the first to seventh disclosures.

Incidentally, in a case in which the core wire is formed to be thick in the overlapping range where the core wire and the inner tube overlap each other in the axial line direction, a space in which the inner tube can be displaced inside (lumen) the outer tube is significantly limited, and the inner tube is displaced along the limited space. Therefore, the inner tube is considered to be more likely to be entangled with the core wire. Thus, in view of such a point, according to the present disclosure, the tapered region narrowed toward the tip end is provided on the tip-end side of the core wire, and the tapered region is disposed over the entire overlapping range of the core wire and the inner tube. In this case, the core wire is formed to be thin in the overlapping range, and it is thus possible to secure a degree of freedom in displacement to which the inner tube can be displaced inside the outer tube. Therefore, it is possible to make the inner tube less likely to be entangled with the core wire.

### Brief Description of Drawings

The aforementioned object and other objects, features, and advantages of the present disclosure will become more apparent from the following detailed description with reference to the accompanying drawings.
[Figure 1] Figure 1 is a schematic overall side view illustrating a configuration of a balloon catheter.
[Figure 2] Figure 2(a) is a vertical sectional view illustrating a configuration of the balloon catheter, (b) is a sectional view along the line A-A in (a), (c) is a sectional view along the line B-B in (a), and (d) is a view illustrating the region C in (a) in an enlarged manner.
[Figure 3] Figure 3 is a side view illustrating a configuration of a core wire.
[Figure 4] Figure 4 is a diagram illustrating a hole portion provided in a wall portion according to another embodiment.

### Description of Embodiments

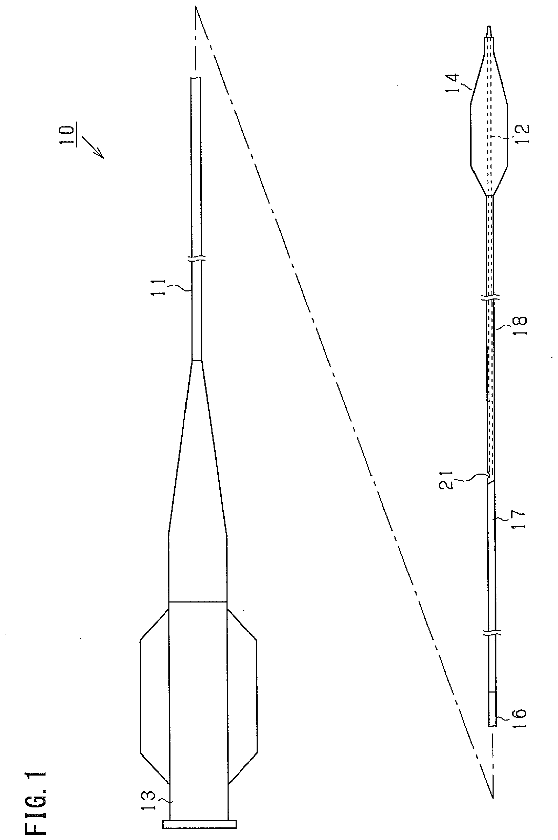

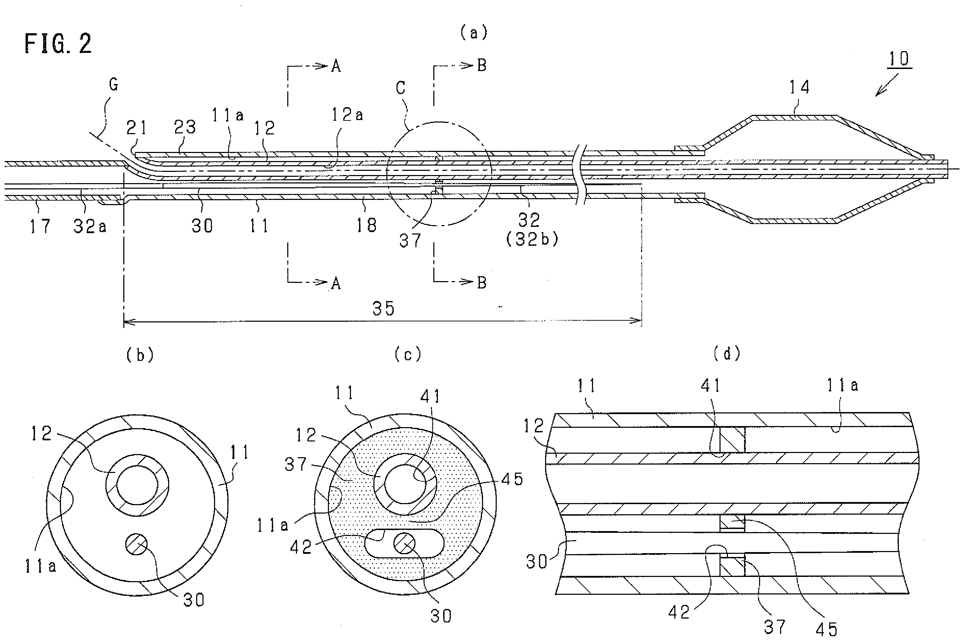

Hereinafter, an embodiment to implement the present disclosure will be described with reference to the drawings. In the present embodiment, a balloon catheter including a balloon that can expand and contract is implemented. Specifically, a PTCA (percutaneous transluminal coronary angioplasty) balloon catheter used for the PTCA is implemented. Hereinafter, a configuration of the balloon catheter will be described on the basis of Figures 1 and 2. Note that Figure 1 is a schematic overall side view illustrating the configuration of the balloon catheter. In Figure 2, (a) is a vertical sectional view illustrating the configuration of the balloon catheter, (b) is a sectional view along the line A-A in (a), (c) is a sectional view along the line B-B in (a), and (d) is a view illustrating the region C in (a) in an enlarged manner.

As illustrated in Figure 1, a balloon catheter 10 includes an outer tube 11, an inner tube 12 inserted into the outer tube 11, a hub 13 attached to a base-end portion (proximal end portion) of the outer tube 11, and a balloon 14 attached to a tip-end side (distal end portion side) of each of the tubes 11 and 12.

The outer tube 11 includes a lumen 11a (see Figure 2(a)) extending over the entire range in the axial line direction therein. Note that the lumen 11a corresponds to the "lumen". The outer tube 11 is configured by a plurality (three in the present embodiment) tubes 16 to 18 aligned in the axial line direction being joined to each other. These tubes 16 to 18 are a base-end-side tube 16, an intermediate tube 17, and a tip-end-side tube 18 in order from the base-end side. The base-end-side tube 16 is formed of metal such as an Ni-Ti alloy or stainless steel and has a base-end portion joined to the hub 13. The intermediate tube 17 is formed of a thermoplastic polyamide elastomer and has lower rigidity than the base-end-side tube 16. The tip-end-side tube 18 is formed of a thermoplastic polyamide elastomer and has lower rigidity than the intermediate tube 17.

The inner tube 12 includes a lumen 12a (see Figure 2(a)) extending over the entire range in the axial line direction therein. The lumen 12a is used as a guide wire lumen into which a guide wire G is inserted. The inner tube 12 is inserted into the tip-end-side tube 18 of the outer tube 11. The inner tube 12 has a base-end portion joined to an intermediate position of the outer tube 11 in the axial line direction, and specifically, the base-end portion is joined to a boundary portion between the intermediate tube 17 and the tip-end-side tube 18.

In this manner, this balloon catheter 10 has a double-tube structure of the tubers 11 and 12 by the inner tube 12 being inserted to the inside (lumen 11a) of the outer tube 11.

A guide wire port 21 is formed at a part of the outer tube 11 at which the outer tube 11 is joined to the inner tube 12. As illustrated in Figure 2(a), the guide wire port 21 is formed to penetrate through a peripheral wall portion 23 surrounding the lumen 11a in the outer tube 11. Specifically, the guide wire port 21 is formed at the base-end portion of the tip-end-side tube 18 and penetrates through the peripheral wall portion 23 of the tip-end-side tube 18.

The base-end-portion of the inner tube 12 is joined to a peripheral edge portion of the guide wire port 21 in the outer tube 11 (the tip-end-side tube 18). In this case, the lumen 12a of the inner tube 12 is opened to the outside of the catheter 10 at the base end thereof via the guide wire port 21. In this manner, the guide wire G introduced from a tip-end opening of the inner tube 12 into the lumen 12a can be guided from the lumen 12a to the outside through the guide wire port 21. In other words, this balloon catheter 10 is an RX-type catheter in which the guide wire G is guided out from a midpoint position in the axial line direction.

A part of the inner tube 12 extends on the tip-end side beyond the outer tube 11, and the balloon 14 is provided to cover the extending region from the outside. The balloon 14 is formed of a thermoplastic polyamide elastomer. However, the balloon 14 may be formed of another thermoplastic resin such as polyethylene or polypropylene.

The balloon 14 has a base-end portion joined to the tip-end portion of the outer tube 11 and a tip-end portion joined to the tip-end portion of the inner tube 12. The inside of the balloon 14 communicates with the hub 13 via the lumen 11a of the outer tube 11, and a compressed fluid supplied via the hub 13 is supplied to the balloon 14 through the lumen 11a. In this case, the lumen 11a is a fluid lumen for allowing the compressed fluid to be distributed therethrough. The balloon 14 is brought into an expanding state when the compressed fluid is supplied to the balloon 14 through the lumen 11a of the outer tube 11, and the balloon is brought into a contracting state when a negative pressure is applied to the lumen 11a and the compressed fluid is discharged.

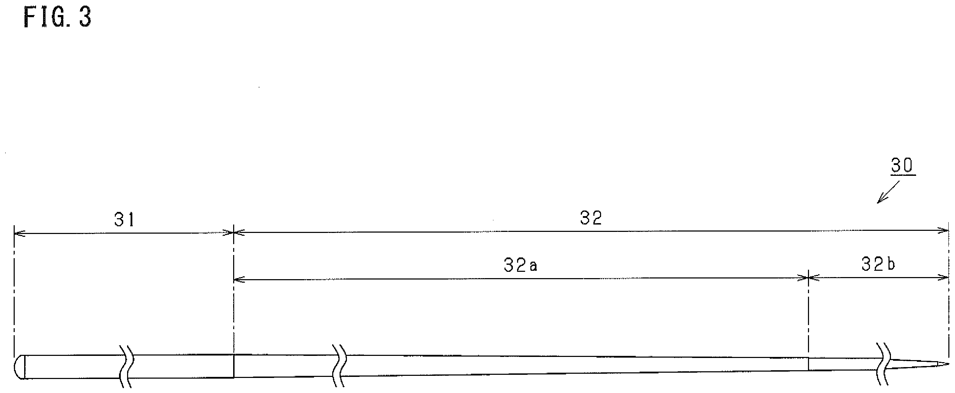

A core wire 30 is provided inside (lumen 11a) the outer tube 11. The core wire 30 is provided for the purpose of enhancing rigidity of the balloon catheter 10, for example, and hereinafter, a configuration regarding the core wire 30 will be described using Figure 3 in addition to Figure 2. Note that Figure 3 is a side view illustrating the configuration of the core wire 30.

As illustrated in Figures 2(a) and (b), the core wire 30 is inserted into the lumen 11a of the outer tube 11. The core wire 30 is formed into a linear shape using a metal material and is formed of stainless steel, for example. The core wire 30 has a horizontal section (a section that perpendicularly intersects the axial line direction) with a circular shape over the entire range in the axial line direction.

The core wire 30 is secured, at the base-end portion thereof, to the hub 13. Specifically, only the base-end portion of the core wire 30 is secured, and the tip-end side beyond the portion is in a non-secured state over the entire range. The core wire 30 extends on the tip-end side beyond the base-end portion (in other words, the guide wire port 21) of the inner tube 12, and the tip-end portion thereof is located in the vicinity of the balloon 14. In this manner, according to the balloon catheter 10, rigidity is applied up to the vicinity of the balloon 14 by the core wire 30, and transmissibility of a force when the balloon catheter 10 is introduced into a body is enhanced.

The core wire 30 is narrowed toward the tip-end side as illustrated in Figure 3. The core wire 30 includes a constant region 31 provided on the base-end side and a tapered region 32 provided on the tip-end side. The constant region 31 has a constant outer diameter (thickness) over the entire range in the axial line direction, and the outer diameter in the tapered region 32 over the entire range in the axial line direction successively decreases from the base-end side toward the tip-end side. Thus, the tapered region 32 has a tapered shape narrowed toward the tip-end side.

More specifically, the tapered region 32 includes a plurality of regions 32a and 32b with different tapering angles (specifically, inclination angles of outer circumferential surfaces relative to the axial line direction). In the present embodiment, the region 32a on the base-end side has a steeper tapering angle than the region 32b on the tip-end side. Therefore, the tapered region 32 is tapered in the two levels in the present embodiment.

Note that the tapered region 32 is not necessarily tapered in the two levels and may be tapered in three or more levels. In other words, the tapered region 32 may be formed to include three or more regions with different tapering angles. Also, the tapered region 32 may be formed at the same tapering angle over the entire range in the axial line direction.

As illustrated in Figure 2(a), the base-end portion of the core wire 30 in the tapered region 32 is located on the base-end side beyond the guide wire port 21 in a state in which the core wire 30 is inserted to the inside of the outer tube 11. Specifically, the boundary portion of each of the regions 32a and 32b in the tapered region 32 is located at substantially the same position as that of the guide wire port 21 in the axial line direction, and more specifically, the boundary portion is located slightly on the tip-end side beyond the guide wire port 21.

Here, in the aforementioned configuration in which the core wire 30 extends on the tip-end side beyond the base-end portion of the inner tube 12 inside the outer tube 11, the extending portion of the core wire 30 is disposed to be aligned with the inner tube 12 inside the outer tube 11. In this case, a situation in which the inner tube 12 and the core wire 30 are entangled with each other when the balloon catheter 10 is introduced into a body is assumed, and there is thus a concern that a problem such as degradation of insertability of the guide wire G into the inner tube 12 may occur. Thus, this balloon catheter 10 is provided with a characteristic configuration in order to restrain the inner tube 12 and the core wire 30 from being entangled in view of such a point. Hereinafter, the characteristic configuration will be described.

As described above, the core wire 30 extends on the tip-end side beyond the base-end portion of the inner tube 12. In this case, an overlapping range 35 in which the core wire 30 and the inner tube 12 overlap each other in the axial line direction is present inside the outer tube 11. The tapered region 32 of the core wire 30 is disposed over the entire overlapping range 35 in the axial line direction.

As illustrated in Figures 2(c) and (d), the outer tube 11 (specifically, the tip-end-side tube 18) is provided with a wall portion 37 to section the lumen 11a in the axial line direction. The wall portion 37 is disposed at an intermediate position of the overlapping range 35 in the axial line direction. Specifically, the wall portion 37 is disposed near the center of the overlapping range 35 in the axial line direction, and more specifically, at the center portion. Also, the outer tube 11 is provided with only one wall portion 37. Note that in Figure 2(c), the wall portion 37 is dot-hatched in the illustration for convenience.

The wall portion 37 is formed of the same material as the material of the tip-end-side tube 18, that is, a thermoplastic polyamide elastomer. The wall portion 37 is formed into a circular shape with substantially the same size as the size of the horizontal section (circular section) of the tip-end-side tube 18 and is joined (secured) through welding (thermal welding) to the tip-end-side tube 18 over the entire peripheral edge portion thereof. In this case, the wall portion 37 is disposed in a state in which the thickness direction is directed in the axial line direction. Also, the wall portion 37 is formed to have a thicker thickness than the thickness of the peripheral wall portion 23 of the outer tube 11. In the present embodiment, the thickness of the wall portion 37 is about two to three times the thickness of the peripheral wall portion 23. Note that the wall portion 37 is not necessarily joined to the tip-end-side tube 18 through welding and may be joined by another bonding method such as adhesion.

Note that the tip-end-side tube 18 may be configured to have a plurality of tube parts divided in the axial line direction at the same position as the position of the wall portion 37 for a reason of fabrication or the like. In this case, the tip-end-side tube 18 is configured by the tube parts being joined to each other through adhesion or the like.

A hole portion 41 into which the inner tube 12 is inserted and a hole portion 42 into which the core wire 30 is inserted are formed in the wall portion 37. Both the hole portions 41 and 42 penetrate through the wall portion 37 in the thickness direction and are disposed to be aligned in the radial direction of the outer tube 11.

Out of the hole portions 41 and 42, the hole portion 41 has a circular shape and has substantially the same size as the size of the horizontal section of the inner tube 12. The inner tube 12 is joined (secured) to the wall portion 37 through welding (thermal welding) in a state in which the inner tube 12 is inserted into the hole portion 41. In this case, the inner tube 12 is secured to the outer tube 11 via the wall portion 37 (in other words, by the wall portion 37). Note that in this case, the wall portion 37 corresponds to the securing portion. Also, the inner tube 12 is not necessarily joined to the wall portion 37 through welding and may be joined by another joining method such as adhesion.

The inner tube 12 may be configured to have a plurality of tube parts divided in the axial line direction at the same position as the position of the wall portion 37 for a reason of fabrication or the like. In this case, the inner tube 12 is configured by the tube parts being joined to each other through welding or the like.

As described above, the inner tube 12 is secured, at the base-end portion thereof, to the outer tube 11 and is secured, at the intermediate portion thereof, to the outer tube 11 via the wall portion 37. The inner tube 12 is secured to the outer tube 11 only at the two locations and is not secured to the outer tube 11 in the other range.

The hole portion 42 has a long hole shape that is long in a direction intersecting an alignment direction of the hole portions 41 and 42 (specifically, a direction perpendicularly intersecting the alignment direction). The hole portion 42 has a width (the length in the alignment direction) that is lightly larger than the outer diameter of the core wire 30 and has a length (the length in the perpendicularly intersecting direction) that is slightly larger than the hole diameter of the hole portion 41. The core wire 30 is inserted into the hole portion 42 as described above. In this case, the core wire 30 is not secured to the wall portion 37 in the inserted state. Also, a region where the core wire 30 is not present in the hole portion 42 in the state in which the core wire 30 is inserted, that is, a region that is not used for the insertion of the core wire 30 is a non-insertion region. The non-insertion region is a region through which a compressed fluid flowing through the lumen 11a of the outer tube 11 is allowed to pass.

A part between the hole portions 41 and 42 in the wall portion 37 serves as a sectioning portion 45 that sections the hole portions 41 and 42. The sectioning portion 45 is interposed between the inner tube 12 inserted into the hole portion 41 and the core wire 30 inserted into the hole portion 42. In this case, the inner tube 12 and the core wire 30 are separated from each other by the sectioning portion 45. Note that the sectioning portion 45 corresponds to the separating portion.

Next, a method of using the balloon catheter 10 will be briefly described.

First, a guiding catheter is inserted into a sheath introduce inserted into a blood vessel, then the guide wire G is inserted into the guiding catheter, and the guide wire G is introduced to a position exceeding a narrow segment.

Next, the guide wire G is inserted into the lumen 12a of the inner tube 12 in the balloon catheter 10. Then, the balloon catheter 10 is introduced into the guiding catheter (and thus a body) with the balloon catheter caused to follow the guide wire G in the inserted state, and the balloon 14 is disposed at the narrow segment in the body.

Here, the inner tube 12 is secured to the outer tube 11 via the wall portion 37 as described above in the balloon catheter 10. Therefore, it is possible to restrain displacement of the inner tube 12 inside (lumen 11a) the outer tube 11 when the balloon catheter 10 is introduced into the body. In this manner, it is possible to restrain the inner tube 12 and the core wire 30 from being entangled with each other inside the outer tube 11. Therefore, it is possible to restrain disadvantages such as degradation of insertability of the guide wire G into the inner tube 12 and collapsing of rigidity balance in the balloon catheter 10, for example.

After the balloon 14 is disposed at the narrow segment, a compressed fluid is supplied from the side of the hub 13 to the balloon 14 via the lumen 11a of the outer tube 11 using a pressurizer. In this manner, the balloon 14 expands, and the expanding balloon 14 extends the narrow segment.

Note that although the balloon catheter 10 is mainly inserted into a blood vessel as described above and is used to treat the inside of the blood vessel, it is also possible to apply the balloon catheter 10 to a "tract" in a biological body other than a blood vessel, such as a urinary tract or a digestive tract, or a "body cavity".

As described above, according to the configuration of the present embodiment described in detail, the following excellent advantages are obtained.

Since only one of the inner tube 12 and the core wire 30 (specifically, the inner tube 12) is secured to the outer tube 11 in the overlapping range 35 in which both the components 12 and 30 overlap each other, a degree of freedom in displacement of the other one (specifically the core wire 30) inside the outer tube 11 is secured. In this case, it is possible to obtain the aforementioned advantage that the tangling of the inner tube 12 and the core wire 30 is restrained while degradation of followability when the balloon catheter 10 is inserted into a curved blood vessel is restrained.

Specifically, the core wire 30 with high rigidity is not secured to the outer tube 11, and only the inner tube 12 with low rigidity is secured to the outer tube 11, out of the inner tube 12 and the core wire 30. In this case, it is possible to suitably restrain the degradation of followability when the balloon catheter 10 is inserted into a curved blood vessel.

The wall portion 37 is provided to section the lumen 11a of the outer tube 11 in the axial line direction, and the wall portion 37 is provided with the hole portion 41 into which the inner tube 12 is inserted and the hole portion 42 into which the core wire 30 is inserted. Also, the inner tube 12 is secured to the outer tube 11 via the wall portion 37, and the core wire 30 is not secured to the wall portion 37, out of the inner tube 12 and the core wire 30. In this case, the sectioning portion 45 between the hole portions 41 and 42 in the wall portion 37 is interposed between the inner tube 12 and the core wire 30 and serves as the separating portion separating both the components 12 and 30. In this manner, it is possible to separate the inner tube 12 and the core wire 30 from each other in addition to securing the inner tube 12 to the outer tube 11 by the wall portion 37. Therefore, it is possible to further restrain both the components 12 and 30 from being entangled with each other. In this case, since the role of the securing portion securing the inner tube 12 and the role of the separating portion separating the inner tube 12 from the core wire 30 are realized by the wall portion 37, it is possible to obtain the aforementioned advantage with a relatively simple configuration.

Since the hole portion 42 provided in the wall portion 37 has a long hole shape, it is possible to widely secure the non-insertion region that is not used for the insertion in the hole portion 42 in a state in which the core wire 30 is inserted into the hole portion 42. In this case, it is possible to use the non-insertion region to allow the compressed fluid to pass therethrough, there is thus no need to separately provide, in the wall portion 37, a hole portion for a fluid to allow the compressed fluid to pass therethrough, and it is possible to simplify the configuration of the wall portion 37.

Since it is necessary to firmly secure the inner tube 12 out of the inner tube 12 and the core wire 30 to the wall portion 37 in a state in which the inner tube 12 is inserted into the hole portion 41, it is desirable for the shape of the hole portion 41 to be a circular shape in accordance with the horizontal sectional shape of the inner tube 12. On the other hand, the core wire 30 is not secured to the wall portion 37, and it is thus not necessary to form the hole portion 42 into such a shape. Thus, in view of such a point, only the hole portion 42 into which the core wire 30 is inserted is formed into the long hole shape out of the hole portions 41 and 42 in the aforementioned embodiment. In this case, it is possible to achieve a configuration that is preferable in practice to form the hole portion into the long hole shape and allow the fluid to pass therethrough.

The tapered region 32 narrowed toward the tip end is provided on the tip-end side of the core wire 30, and the tapered region 32 is disposed over the entire overlapping range 35 of the core wire 30 and the inner tube 12. In this case, the core wire 30 is narrowed in the overlapping range 35, and it is thus possible to secure a degree of freedom in displacement to which the inner tube 12 can be displaced inside the outer tube 11. Therefore, it is possible to make the inner tube 12 less likely to be entangled with the core wire 30.

The present disclosure is not limited to the aforementioned embodiment and may be performed as follows, for example.

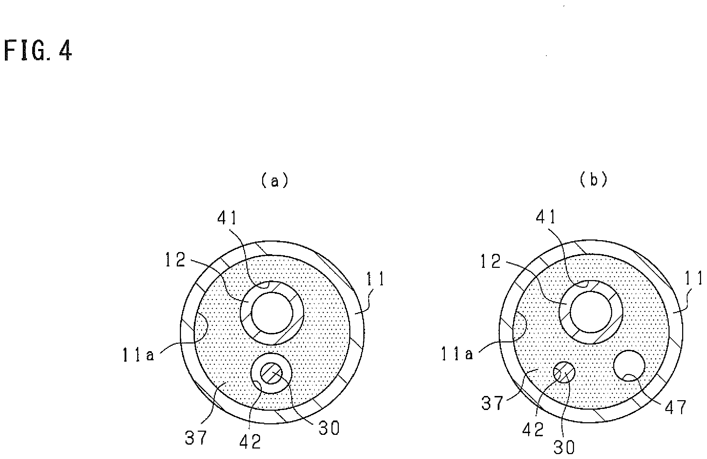

(1) Although the inner tube 12 is secured to the outer tube 11 by the wall portion 37 in the aforementioned embodiment, the configuration for securing the inner tube 12 to the outer tube 11 is not necessarily limited thereto. For example, the inner tube 12 may be secured to the outer tube 11 through welding or adhesion. In this case, a welded part or an adhesion layer interposed between the inner tube 12 and the outer tube 11 corresponds to the securing portion.
(2) Although the wall portion 37 is disposed at the center portion of the overlapping range 35 in the aforementioned embodiment, the wall portion 37 may be disposed on the base-end side or the tip-end side in the overlapping range 35. Also, although only one wall portion 37 is provided in the aforementioned embodiment, a plurality of wall portions 37 may be provided at predetermined intervals.
(3) Although the inner tube 12 and the core wire 30 are separated by the sectioning portion 45 of the wall portion 37 in the aforementioned embodiment, the separating portion separating both the components 12 and 30 is not necessarily formed by the wall portion 37. For example, a linear member extending through a part between the inner tube 12 and the core wire 30 may be provided inside the outer tube 11, and the linear member may be caused to serve as the separating portion. In this case, forming the linear member using a resin material and securing each of the opposite end portions thereof to the outer tube 11 is conceived. An application of such a configuration in the configuration in (1) described above, for example, is conceived. In this case, the separating portion may be disposed at the same position as the position of the securing portion in the axial line direction or may be disposed at a different position.
(4) Although the inner tube 12 is secured to the outer tube 11 by the wall portion 37 out of the inner tube 12 and the core wire 30 in the aforementioned embodiment, this may be changed, and the core wire 30 may be secured to the outer tube 11 by the wall portion 37. It is possible to restrain the inner tube 12 and the core wire 30 from being entangled in this case as well.
(5) Although the hole portion 42 is formed into the long hole shape out of the hole portions 41 and 42 provided in the wall portion 37 in the aforementioned embodiment, the hole portion 41 may be formed into a long hole shape instead of or in addition to the hole portion 42. Even if the hole portion 41 is formed into the long hole shape, it is possible to secure the non-insertion region into which the inner tube 12 is not inserted in the hole portion 41 and thereby to use the non-insertion region to allow the fluid to pass therethrough.
(6) Although the hole portion 42 is formed in to the long hole shape in the aforementioned embodiment, this may be changed, and the hole portion 42 may be formed into a circular shape (round hole shape) as illustrated in Figure 4(a). It is possible to secure the non-insertion region into which the core wire 30 is not inserted in the hole portion 42 by forming the diameter of the hole portion 42 to be larger than the outer diameter of the core wire 30 in this case as well. It is thus possible to allow the fluid to be distributed through the non-insertion region. Also, as illustrated in Figure 4(b), a dedicated hole portion 47 for causing the fluid to pass therethrough may be provided in the wall portion 37 in addition to the hole portions 41 and 42.
(7) Although the case in which the present disclosure is applied to an RX-type balloon catheter has been described in the aforementioned embodiment, the present disclosure may be applied to other RX-type catheters. In other words, it is possible to apply the present disclosure to any configuration as long as a core wire is inserted to the inside of an outer tube in an RX-type catheter.

Although the present disclosure has been described in accordance with the embodiment, the present disclosure is to be understood as not being limited to the embodiment and the structure. The present disclosure also includes various modification examples and modifications within an equivalent scope. In addition, various combinations and forms and yet other combinations and forms adding only one element or more or less elements thereto are also included within the range and the technical scope of the present disclosure.

### Reference Signs List

- 10: Balloon catheter
- 11: Outer tube
- 12: Inner tube
- 14: Balloon
- 30: Core wire
- 32: Tapered region
- 35: Overlapping range
- 37: Wall portion
- 41: Hole portion
- 42: Hole portion
- 45: Sectioning portion serving as separating portion

## Claims

1. A catheter comprising:
an outer tube that includes a lumen therein;
an inner tube that is inserted into the lumen of the outer tube and has a base-end portion joined to an intermediate position of the outer tube in an axial line direction; and
a core wire that is inserted into the lumen of the outer tube and is provided to extend on a tip-end side beyond the base-end portion of the inner tube,
wherein a securing portion that secures the inner tube or the core wire to the outer tube is provided at an intermediate position of an overlapping range, in which the inner tube and the core wire overlap each other in the axial line direction, in the axial line direction.

2. The catheter according to claim 1, wherein the securing portion is adapted to secure the inner tube to the outer tube.

3. The catheter according to claim 1 or 2, further comprising:
a separating portion that is interposed between the inner tube and the core wire to separate the inner tube and the core wire.

4. The catheter according to claim 3, further comprising:
a wall portion that is provided to section the lumen in the axial line direction,
wherein a hole portion into which the inner tube is inserted and a hole portion into which the core wire is inserted are formed in the wall portion,
any one of the inner tube and the core wire is secured to the outer tube via the wall portion serving as the securing portion while the other one of the inner tube and the core wire is not secured to the wall portion, and
the separating portion is formed by a part between the hole portions in the wall portion.

5. The catheter according to claim 4, wherein the inner tube as the one of the inner tube and the core wire is secured to the outer tube via the wall portion while the core wire as the other one of the inner tube and the core wire is not secured to the wall portion.

6. The catheter according to claim 4 or 5,
wherein the lumen is a fluid lumen through which a fluid flows, and
the hole portions have long hole shapes.

7. The catheter according to claim 6, wherein the hole portion into which the other one of the inner tube and the core wire that is not secured to the wall portion is inserted out of the hole portions has a long hole shape.

8. The catheter according to any one of claims 1 to 7,
wherein the core wire has, on a tip-end side, a tapered region that is formed into a tapered shape to be narrowed toward the tip end, and
the tapered region is disposed over the entire overlapping range.
